Europäisches Patentamt

European Patent Office

Office européen des brevets

(19)

(11) Publication number: **0 074 244**
A1

(12) # EUROPEAN PATENT APPLICATION

(21) Application number: **82304597.6**

(22) Date of filing: **01.09.82**

(51) Int. Cl.³: **C 12 P 19/62**
//(C12P19/62, C12R1/465,
C07H17/08)

(30) Priority: **04.09.81 JP 138365/81**

(43) Date of publication of application:
**16.03.83 Bulletin 83/11**

(84) Designated Contracting States:
**DE FR GB**

(71) Applicant: KYOWA HAKKO KOGYO CO., LTD.
Ohtemachi Bldg., 6-1 Ohtemachi Itchome
Chiyoda-ku Tokyo 100(JP)

(72) Inventor: Sakurai, Minoru
2-12-306, Kyowa-cho Hofu-shi
Yamaguchi-ken(JP)

(72) Inventor: Kawahara, Shin
2-3-107, Kyowa-cho Hofu-shi
Yamaguchi-ken(JP)

(72) Inventor: Hagihara, Takeshige
1-2, Kyowa-cho Hofu-shi
Yamaguchi-ken(JP)

(74) Representative: Lambert, Hugh Richmond et al,
D. YOUNG & CO. 10 Staple Inn
London, WC1V 7RD(GB)

(54) Process for producing spiramycin I.

(57) A process for producing the antibiotic Spiramycin factor
I substantially free of Spiramycin factors II and III by culturing
a mutant microorganism belonging to the genus *Streptomy-
ces*, and in particular Streptomyces ambofacience CS-93,
NRRL 12511, which mutant lacks the ability to produce
Spiramycin factors II and III. The antibiotic is recovered from
the culture liquor.

EP 0 074 244 A1

-1-

## PROCESS FOR PRODUCING
## SPIRAMYCIN I

The present invention pertains generally to a process for producing the known antibiotic substance Spiramycin factor I and, more specifically, to an improved process for producing Spiramycin factor I substantially free of other Spiramycin factors.

Spiramycin is a known antibacterial classified in the erythromycin-carbomycin group, and is comprised of Spiramycin factors I, II, and III. (The Merck Index, Ninth ed.) These Spiramycin antibiotics are known to be concomitantly produced by culturing Streptomyces ambofacience NRRL 2420 (Japanese Patent Publication No. 499/59), and Spiramycin I is known to show the highest biological activity among the three Spiramycin factors (French Pharmacopoeia).

However, the known processes for producing Spiramycin provide a mixture of Spiramycin factors I, II, and III in the culture liquor. Spiramycin I usually accounts for about 60% of the whole product. In order to produce only Spiramycin I, the other factors must be removed from the mixture (Japanese Patent Publication Nos. 4599/61 and 21546/61). However, removal of Spiramycin factors II and III to purify and isolate Spiramycin I is industrially quite difficult due to the extremely analogous chemical structures of the three factors. Accordingly, a need exists for a process whereby Spiramycin factor I is produced free of other Spiramycin factors in an economical and industrially feasible manner. To this end, a mutant microorganism strain has been obtained from a Spiramycin factors I, II and III producing microorganism, which mutant lacks the ability to produce Spiramycin factors II and III but produces, upon fermentation, Spiramycin factor I in high yield.

In accordance with the present invention a mutant strain of microorganism is obtained from a Spiramycin factor producing parent strain, e.g. Streptomyces ambofacience NRRL-2420 which mutant is capable of producing Spiramycin factor I but lacks the ability to produce Spiramycin factors II and III.

Also according to the present invention, a process for producing Spiramycin factor I substantially free of other Spiramycin factors comprises culturing a Spiramycin factor I producing mutant microorganism, which mutant lacks the ability to produce Spiramycin factors II and III, in a nutrient medium until recoverable quantities of Spiramycin factor I are produced and thereafter recovering Spiramycin factor I therefrom.

Finally, the present invention includes a biologically pure culture of a cerulenin resistant mutant microorganism having the identifying characteristics of Streptomyces ambofacience CS-93, NRRL 12511, which culture produces, upon fermentation, recoverable quantities of Spiramycin factor I without substantial concomitant production of Spiramycin factors II and III.

In accordance with the present invention, a Spiramycin producing parent strain is first mutated to produce a mutant which lacks the ability to produce Spiramycin factors II and III but retains the ability to produce Spiramycin factor I. As the parent strain, Streptomyces ambofacience NRRL 2420 is used as a known Spiramycin producing strain.

As an example of the mutation inducing method, Streptomyces ambofacience NRRL 2420 is treated with 1 mg/ml of N-methyl-N'-nitroso-N-nitroguanidine at 28°C for 45 minutes in a tris.maleate buffer solution (pH 9.0), and then inoculated on an agar medium having the composition given in Table 1. After culturing at 28°C for 5 to 10 days, living colonies are picked up to examine ability to produce Spiramycin factor I using a medium for producing Spiramycin having the composition described hereinafter. In such manner Streptomyces ambofacience CS-93 is isolated as a strain having the ability to produce Spiramycin factor I without substantial concomitant production of Spiramycin factors II and III. This strain has been deposited under the conditions of the Budapest Treaty with the Agricultural Research Culture Collection, Peoria, Illinois, under accession number NRRL 12511.

- 4 -

Table 1

| Maltose | 10 g/ℓ |
|---|---|
| Dipotassium hydrogen phosphate | 0.5 g/ℓ |
| Sodium chloride | 0.1 g/ℓ |
| Iron sulfate | 0.1 g/ℓ |
| Bacto-tryptone | 5 g/ℓ |
| Cerulenin | 50 mg/ℓ |
| Agar | 20 g/ℓ |
| pH | 7.4 (NaOH) |

The mutant strain of the present invention is resistant to cerulenin as shown by the following experiment.

Experiment:

Each test microorganism is inoculated into a 250 ml-flask containing 10 ml of a medium comprising the ingredients of Table 1 except agar, and cultured at 28°C for 24 hours with shaking. The degree of growth is determined by measuring the absorbancy at 660 nm, and the results are shown in Table 2 as relative values.

Table 2

| Strain | Degree of growth (relative value) | |
|---|---|---|
| | Medium containing no cerulenin | Medium containing 50 mg/ℓ cerulenin |
| NRRL 2420 | 100 | 0 |
| NRRL 12511 (CS-93) | 100 | 50 |

- 5 -

The medium for culturing the microorganism having an ability to produce Spiramycin I alone is an ordinary liquid medium containing a carbon source, nitrogen source, inorganic salts and, if necessary, trace amounts of other nutrients. As the carbon source, monosaccharides such as glucose, polysaccharides such as starch, sugar alcohols such as glycerol, and oils such as soybean oil may be used. As the nitrogen source, ammonium salts such as ammonium chloride and ammonium sulfate, and organic nitrogen sources such as corn steep liquor and baker's yeast are used. Culturing is preferably conducted under aerobic conditions. The pH of the medium is adjusted to substantial neutrality, preferably 6 to 8, before sterilization. Culturing temperature is 24 to 30°C. After culturing for 5 to 7 days under the above-mentioned conditions, a remarkable amount of Spiramycin I is accumulated in the culture liquor, and neither Spiramycin factor II nor III is detected by bioautography described hereinafter.

Spiramycin I is readily recovered from the culture liquor, for example, by removing cells, extracting Spiramycin I with a water-immiscible solvent, and concentrating the extract, followed by crystallization.

While the mutation inducing treatment described above employs of particular parent strain and mutagenic agent, those skilled in the art will appreciate that other mutagens such as X-ray irradiation, ultraviolet irradiation or mutation inducing chemicals may be employed as well as other parent strains so long as such strain normally elaborates the Spiramycin factors. In like manner, mutants derived from such other parent strains belonging to the genus Streptomyces and which have the ability to produce Spiramycin factor I without substantial concomitant production of Spiramycin factors II and III are included within the present invention.

Certain specific embodiments of the present invention are illustrated by the following representative examples.

## Example 1

In this example, _Streptomyces ambofacience_ CS-93 (NRRL 12511) is inoculated into a 2 ℓ-flask containing 300 ml of the seed medium shown in Table 3, and cultured at 28°C for 48 hours with shaking. Then 1 ml of the resulting culture liquor is added to a 250 ml-flask containing 30 ml of the main fermentation medium shown in Table 3, and cultured at 28°C for 7 days. As a control, the same procedure as above is repeated using _Streptomyces ambofacience_ NRRL 2420 in place of the NRRL 12511 strain.

## Table 3

| Seed Medium | | Main Fermentation Medium | | |
|---|---|---|---|---|
| Corn steep liquor | 40 g/ℓ | Starch | 40 | g/ℓ |
| Glucose | 20 g/ℓ | $NH_4Cl$ | 5 | g/ℓ |
| NaCl | 5 g/ℓ | NaCl | 20 | g/ℓ |
| $MgSO_4.7H_2O$ | 1 g/ℓ | $MgSO_4.7H_2O$ | 1 | g/ℓ |
| $CaCO_3$ | 5 g/ℓ | $KH_2PO_4$ | 2 | g/ℓ |
| Soybean oil | 4 ml/ℓ | $ZnSO_4.7H_2O$ | 5 | mg/ℓ |
| pH 7.4 (NaOH) | | $CoCl_2.6H_2O$ | 0.3 | mg/ℓ |
| sterilized at 120°C for 20 min. | | pH 6.8 (NaOH) | | |
| | | Soybean oil | 4 | ml/ℓ |
| | | $CaCO_3$ | 5 | g/ℓ |
| | | sterilized at 120°C for 20 min. | | |

After completion of fermentation, the presence of each Spiramycin factor in the culture liquor is determined according to bioautography by bioassay using Bacillus subtilis ATCC 6633 as a test microorganism, and the results are shown in Table 4. The bioassay is conducted in a known manner (Japanese Patent Publication No. 349/61). That is, Spiramycin factors are extracted from the culture liquor with butyl acetate. Then, 5 µl of the extract is spotted on a filter paper and subjected to paper chromatography [solvent : cyclohexane : methyl ethyl ketone : water = 17 : 3 : 5 (v/v), about 3.5 hours].

Separately, the medium containing Bacillus subtilis ATCC 6633 is put into a glass tray and allowed to coagulate. The filter paper treated as above is put on the glass tray and kept there for 30 minutes. After culturing at 30°C for 16 hours, inhibition zone is observed on the glass tray. The glass tray is then put on a printing paper and a photograph thereof is taken. From the photograph, the area of the inhibition zone is measured and Spiramycin factors in the culture liquor are determined based on the standard curve obtained with known amounts of Spiramycin factors I, II and III. The detection limit of this method is 0.01 mcg and, in the case where no inhibition zone is observed, the relative content is not more than 1%.

Thus, 1 liter of the culture liquor of the CS-93 strain is filtered to remove the cells, and the resulting filtrate is made weakly alkaline (pH 9.5), and extracted with 500 ml of methyl isobutyl ketone. To this extract 500 ml of phosphoric acid-acidic water (pH 3.0) is added to extract Spiramycin factors. The aqueous extract is rendered weakly alkaline (pH 9.5), and again extracted with 100 ml of chloroform. The chloroform extract is concentrated, followed by crystallization to obtain 160 mg of Spiramycin crystals. The crystals show a single spot in the aforesaid bioautography, and the $R_f$ of the spot coincides with that of Spiramycin I. Thus, it is confirmed that the crystals contain substantially no Spiramycin factors II and III.

The culture liquor of control NRRL 2420 is similarly treated to obtain about 200 mg of Spiramycin crystals. The crystals are separated into three ingredients by the bioautography, and the $R_f$ values of the three ingredients coincide with those of Spiramycin factors I, II and III, respectively. The composition ratio of the three ingredients substantially coincide with that of the culture liquor.

## Table 4

| Strain | Biological Potency (mcg/ml) | Spiramycin Ratio (%) | | |
|---|---|---|---|---|
| | | I | II | III |
| NRRL 2420 | 800 | 65 | 20 | 15 |
| NRRL 12511 (CS-93) | 1000 | 100 | 0 | 0 |

## Example 2

In this example, 3 ml each of a culture liquor of the NRRL 2420 strain and a culture liquor of the mutant strain, CS-93, obtained by the same seed culture as in Example 1, is added to a 2 ℓ-flask containing 100 ml of the main fermentation medium shown in Table 3, and cultured at 28°C for 7 days. After completion of culturing, bioautography is carried out as in Example 1 and the results are shown in Table 5.

## Table 5

| Strain | Biological Potency (mcg/ml) | Spiramycin Ratio (%) | | |
|---|---|---|---|---|
| | | I | II | III |
| NRRL 2420 | 800 | 65 | 20 | 15 |
| CS-93 | 1000 | 100 | 0 | 0 |

One liter of the culture liquor of the CS-93 strain is purified in the same manner as in Example 1 to obtain 160 mg of Spiramycin I crystals.  Bioautography of the crystals shows a single spot, and no spots of Spiramycin factors II and III are observed.

Example 3

In this example, 300 ml each of a culture liquor of the NRRL 2420 strain and a culture liquor of the mutant strain, CS-93, obtained by the same seed culture as in Example 1, is added to a 5 ℓ-glass jar fermenter containing 3 ℓ of the main fermentation medium shown in Table 3, and cultured at 28°C for 7 days with agitation (500 rpm) and aeration (3 ℓ/min).  After completion of culturing, bioautography is carried out as in Example 1 and the results are shown in Table 6.

### Table 6

| Strain | Biological Potency (mcg/ml) | Spiramycin Ratio (%) | | |
|---|---|---|---|---|
| | | I | II | III |
| NRRL 2420 | 900 | 65 | 20 | 15 |
| CS-93 | 1100 | 100 | 0 | 0 |

Three liters of the culture liquor of the CS-93 strain is purified in the same manner as in Example 1 to obtain 500 mg of Spiramycin I crystals. · Bioautography of the crystals shows a single spot, and no spots of Spiramycin factors II and III are observed.  Three liters of the · culture liquor of the NRRL-2420 strain is similarly treated to obtain 630 mg of Spiramycin crystals.  Bioautography of the crystals shows three spots, whereby  the crystals prove to be a mixture of Spiramycin factors I, II and III.

0074244

- 10 -

<u>CLAIMS</u>

1.    A process for producing Spiramycin factor I substantially free of Spiramycin factors II and III which comprises culturing a mutant microorganism belonging to the genus <u>Streptomyces</u> and having the ability to produce Spiramycin factor I without concomitant production of Spiramycin factors II and III, in a nutrient medium until recoverable quantities of Spiramycin factor I are produced in the culture liquor and thereafter recovering Spiramycin factor I therefrom.

2.    A process according to claim 1 wherein said mutant microorganism belongs to the species <u>Streptomyces ambofacience</u>.

3.    A process according to claim 2 wherein said mutant is resistant to cerulenin.

4.    A process according to claim 3 wherein said mutant microorganism is <u>Streptomyces ambofacience</u> CS-93, NRRL 12511.

5.    A process according to claim 1 wherein said culturing step is carried out at a temperature of from 24°C to 30°C for about 5 to 7 days.

6.    A biologically pure culture of a cerulenin resistant mutant microorganism having the identifying characteristics of <u>Streptomyces ambofacience</u> NRRL 12511 which culture when fermented produces recoverable quantities of Spiramycin factor I substantially free of Spiramycin factors II and III.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | C 12 P 19/62 // |
| A | US-A-2 978 380 (J.PREUD'HOMME) *The whole document* | 1,2 | (C 12 P 19/62 C 12 R 1/465 C 07 H 17/08 ) |
| | --- | | |
| A | FR-A-1 211 310 (RHONE) *Pages 1-4,7* | 1,2 | |
| | ----- | | |

|  | TECHNICAL FIELDS SEARCHED (Int. Cl. 3) |
|---|---|
| | C 12 P 19/00 C 12 R 1/00 |

The present search report has been drawn up for all claims

| Place of search THE HAGUE | Date of completion of the search 22-11-1982 | Examiner RAJIC M. |
|---|---|---|